# EUROPEAN PATENT SPECIFICATION

(11) **EP 4 505 943 B1**
(45) Date of publication and mention of the grant of the patent: **23.09.2026**
(21) Application number: 23191117.3
(22) Date of filing: 11.08.2023
(51) Int. Cl.: A61B 6/00, A61B 5/055, A61B 5/00, H05K 1/02, A61B 6/03

(54) **CIRCUIT BOARD FOR AN IMAGING SYSTEM**
LEITERPLATTE FÜR EIN ABBILDUNGSSYSTEM
CARTE DE CIRCUIT IMPRIMÉ POUR UN SYSTÈME D'IMAGERIE

(43) Date of publication of application: 12.02.2025
(73) Proprietor: Siemens Healthineers AG, 91301 Forchheim (DE); Siemens Medical Solutions USA, Inc., Malvern, PA 19355 (US)
(72) Inventor: Eberler, Ludwig, 92318 Neumarkt i.d.OPf. (DE); Judenhofer, Martin, Knoxville, 37922 (US); Loope, Michael, Louisville, 37777 (US); Martius, Sebastian, 91301 Forchheim (DE); Moor, Andrew Philip, Knoxville, 37932 (US); Rösler, Manuela, 91054 Erlangen (DE)
(74) Representative: Siemens Healthineers Patent Attorneys

(56) References cited:
- US-A1- 2012 206 139
- US-A1- 2012 242 339
- US-A1- 2022 099 769

## Description

The invention relates to an imaging system, a circuit board for an imaging system, and a method for designing a circuit board arrangement.

Independent of the grammatical term usage, individuals with male, female or other gender identities are included within the term.

Imaging systems, in which magnetic resonance imaging (MRI) is combined with other imaging modalities, such as with positron emission tomography (PET), provide additional diagnostic possibilities. For example, images with the different modalities may be taken simultaneously or nearly simultaneously and without repositioning a subject, such as a patient, in between taking images. However, MRI systems create magnetic fields, in particular magnetic gradient fields, with high field strength that change quickly over time. Therefore, electrically conductive materials that are positioned within the magnetic field of the MRI system may heat up due to eddy currents that are induced by the rapidly varying magnetic gradient field. On the other hand, in combined imaging systems, due to design requirements such as the patient bore diameter and/or power-to-field efficiency of the gradient coil, electronics of the other imaging modality, e.g. PET electronics, usually including printed circuit boards, often have to be placed close to the gradient coils of the MRI system. In this position, field components of the MRI magnetic gradient field perpendicular to the z-axis of the MRI system are relatively large. Since the PET electronics necessarily comprise conductive materials, such as copper layers, and their design is restricted by requirements of the PET system itself, eddy currents are induced in the PET electronics leading to heating of the electronics. This, in turn, may lead to various problems such as a temperature-dependent drift of the PET signal, a reduction of the lifetime or complete destruction of temperature-sensitive components, a (partial) detachment of components of printed circuit boards, or a requirement to replace detector elements. Furthermore, the induced eddy currents can cause image quality problems such as echo-planar imaging (EPI) ghosting.

There are some measures that may be taken to reduce the heating induced by eddy currents. For example, materials may be used that have a lower electrical conductivity or the orientation of electrically conductive surface may be set to be non-perpendicular to the magnetic gradient field. However, in order to ensure the functionality of the additional imaging system, such as a PET system, the degree of freedom in designing the electronics is limited. For example, the integrity of clock signals must be ensured.

One possible measure is to maximize the impedance in the frequency range of the eddy currents (typically kHz) while, at the same time, keeping it small in the frequency range of the clock (typically in the range of MHz to GHz). For radio frequency shields for birdcage coils this may be achieved by capacitive segmentation of the shield, e.g. with discrete components and/or by laminating capacitances. However, on printed circuit boards, the achievable areal capacitance is limited by the minimum substrate thickness of the dielectric layer and its dielectric constant and additional components, such as additional discrete components, cause additional costs. Furthermore, a practical implementation of these measure requires space, which is limited due to the MRI components.

A magnetic resonance-compatible electrical device with radio frequency shielding is known from US 2012/242339 A1. A hybrid PET/MRI scanner is known from US 2012/206139 A1.

It is an object of the invention to provide a means to reduce the electromagnetic induction of eddy currents by MRI magnetic fields in circuit boards of combined imaging systems, which is preferably cost-efficient and flexible with respect to the design of the circuit boards.

This object is met or exceeded by an imaging system according to claim 1, a circuit board according to claim 10, and a method according to claim 11. Further advantages and features result from the dependent claims, the description and the attached figures.

According to a first aspect of the invention, an imaging system, in particular medical imaging system, is provided. The imaging system comprises a magnetic resonance imaging system with a gradient coil arrangement configured to produce a varying magnetic gradient field during operation. Furthermore, the medical imaging system comprises one or several printed circuit boards arranged such that they are within the magnetic gradient field of the magnetic resonance imaging system during operation, each circuit board having at least one conducting layer, in particular a copper layer, wherein at least one of the circuit boards comprises a mesh of holes that spreads over at least a partial region of the conducting layer. The imaging system may comprise at least one further imaging modality. The one or several printed circuit boards may be part of the at least one further imaging modality. Alternatively, the one or several printed circuit boards may be part of the magnetic resonance imaging system or of any other electronics of the whole imaging system. In one variation, the several circuit boards are part of a stack of circuit boards. Optionally, the at least one circuit board may comprise a stack of layers. Advantageously, the mesh of holes can increase the impedance of the conducting layer, at least in the region of the mesh. The mesh of holes may be configured to increase the impedance of the circuit board in the kHz range. It has been found that this range may be particularly relevant for the purpose of eddy current induced heating due to the changing magnetic fields. In this regard, the mesh of holes may have a similar effect as physically decreasing the thickness of the conducting layer. However, while this "virtual" decrease of thickness can be manufactured relatively easily via the mesh of holes, an actual reduction of the thickness would be more difficult to manufacture. Accordingly, it has been found that a mesh of holes can be an easy to manufacture and surprisingly efficient way of decreasing the induction of heat in the circuit board. Further advantageously, the provision of the mesh does not require additional discrete components or additional space in order to reduce the heating. Furthermore, the arrangement or placement of the mesh can be easily adapted corresponding to the individual needs depending on the function a design of the circuit board. This may for example, be particularly beneficial for designing sensitive circuit areas. The inventive solution may thus provide a flexible and adaptable way of reducing the heat induction.

The one or several printed circuit boards may be part of the electronics of the imaging system. Since providing the mesh of holes can reduce a heating induced by changing magnetic fields, the design of the imaging system and its electronics can be more flexible. Usually, the magnetic resonance imaging system and its magnetic fields restrict the placement of circuit boards or requires measures of shielding the corresponding electronics or other measures to reduce an induction of currents due to the magnetic fields. There often is a competition of trying to place the electronics in an area where the magnetic fields are as low as possible, on the one hand, and, on the other hand, having only limited space available in these areas as well as actually wanting the electronics to be close to a subject or object that is examined for various reasons. Due to the mesh, a greater flexibility can be achieved with regard to the circuit board placement. For example, a more advantageous placement of circuit boards of another imaging modality may become feasible. According to one option, the term "within the magnetic gradient field" may in particular mean, that the position of the circuit board is in an area where, during operation, the magnetic fields or the variation of the magnetic field, is higher than a thousandth, preferably higher than a hundredth, of the maximum value of the magnetic fields or the variation of the magnetic field. The circuit board may be positioned inside the gradient coil of the magnetic resonance imaging system. The circuit board may, for example, be arranged around an examination area, where an object or subject, such as a patient, is to be examined. According to one option, the at least one circuit board is not elastic. For example, the at least one circuit board may have an elasticity value defined by a Young's modulus of more than 15 Gpa, preferably more than 20 Gpa. It may be provided that the circuit board is installed in the imaging system without being flexed or bended for a substantial amount. The printed circuit not being flexed or bended may provide more freedom in designing the circuit board, since, for example, a flexible printed circuit may have to be designed such that a bending is not detrimental to the function of the circuit board.

The conducting layer may comprise a pattern of conductive features, such as traces or lines, planes, or other features. The conductive features may be etched into the conductive layer. The conducting layer may, for example, be a metal layer. Preferably, the conductive layer may be made from copper. The conductive layer may preferably be laminated on a non-conductive layer and/or between two non-conductive layers. The mesh of holes spreads over at least a partial region of the conducting layer. The mesh of holes may, for example, comprise more than 10 holes, preferably more than 100 holes. Optionally, the mesh may spread over the whole conductive layer. Having the mesh spread over a large region or even the whole conductive layer may increase the effect reducing an induction of heat. On the other hand, the mesh spreading only over a partial region may be beneficial for improving the signal quality. For example, depending on the specific signal line, some critical signal lines may be provided that are not leading across the mesh, when it is found that these critical signals are less reliable, or not reliable enough, when they are going across the mesh.

According to an embodiment, the imaging system further comprises a positron emission tomography (PET) system, wherein the at least one circuit board is part of the electronics of the positron emission tomography system. For technical reasons, it is often beneficial to place electronics of the PET system, in particular including printed circuit boards, close to the magnetic coils. Hence, the inductive heating may be particularly relevant for PET systems. Accordingly, the inventive system may be particularly advantageous, when the at least one printed circuit board is part of a PET system. A further particular advantage with respect to this embodiment can be, that the mesh tends to increase the impedance in the kHz range but keep the impedance low enough for PET specific purposes. At least some parts of the PET electronics, usually operate at higher frequencies that are not affected negatively as much. In other words, the impedance of the circuit board due to the mesh may still be low enough at the higher frequencies required for (at least some of) the PET electronics. Hence, there can be a synergy effect of the properties of the PET system and the properties of a printed circuit board having a mesh of holes. While the invention can be particularly advantageous for a PET system, the invention can generally also be used for any other type of electronics. The other type of electronics can be part of the MRI system or of another imaging modality or of any other electronic component.

According to the invention, the at least one circuit board comprises at least one electronic signal line running across the mesh. It has been found that, surprisingly, the impact of the mesh of holes on the signal quality of the signal line can often be low enough that it is still feasible without significantly impeding the overall functionality of the electronics the circuit board is a part of.

According to the invention, the at least one circuit board comprises a trace of conducting material running across the mesh and following the course of the at least one signal line, wherein the trace of conducting material is wider than the at least one signal line, such that, at least across the mesh, the at least one signal line is widened by the trace of conducting material. Via the trace, the effective signal line may be widened. It has been found that a signal quality can be improved considerably with the trace of conducting material while increasing an induced heating only to a small amount. Hence incorrect wave impedances or signal integrity degradation of the corresponding signal line induced by the mesh may be compensated for via the trace of conducting material. The trace of conducting material may in particular be provided for very sensitive signal lines. Preferably, the width of the trace may be 2 to 15 times, more preferably 4 to 8 times, greater than the width of the signal line. It has been found that this ratio can bring good benefits in signal quality, while at the same time keeping the increased induction of heat relatively small. The conducting material of the trace may preferably be a metal material, more preferably copper.

According to an embodiment, at least one signal line which is widened by the trace of conducting material is a signal line for a clock signal. The integrity of clock signals is usually vital, in particular for the purpose of PET electronics. Generally, the frequency of clock signals in PET electronics is in the order of magnitude of MHz to GHz. The mesh tends to increase the impedance in the kHz range while an increase in impedance can be relatively small for higher frequencies, i.e. in the range of MHz to GHz. Hence the mesh may be particularly suitable even for sensible clock signal lines. However, the integrity of the clock signal lines running over the mesh can be improved even further by the trace of conducting material. Accordingly, it may be possible to achieve reliable clock signal lines even on the mesh. While the widening of conducting material by the trace can be particularly advantageous for a clock signal line, the widening of conducting material by the trace can generally also be used for any other type of signal line. The circuit board does not have to have this feature for a clock signal. In some embodiments the mesh may not extend to the area of the clock signal line. In some embodiments, the clock signal line might work being on the mesh without the widened trace.

According to an embodiment, the cross-sectional size of the mesh's holes is 10 to 1000 micrometres, preferably 70 to 500 micrometres, more preferably 100 to 250 micrometres. It has been found that such a size can be advantageous in giving a particularly high impedance at the frequency relevant the induction of eddy currents due to the changing magnetic fields of the MRT system (i.e. in the kHz range), while, at the same time, increasing the impedance only a little for higher frequencies, e.g. in the range of MHz or GHz. Hence, the impedance can still be low enough, and thus viable for the purposes of the circuit board, in this range. 100 to 250 micrometres can be particularly well-suited not to decrease the signal quality of signal lines that are on the mesh too much. The holes may be circular. In this case the cross-section size may be the diameter. The holes may be quadratic. In this case the cross-section size may be the length of one side. The holes may be of another shape, e.g. hexagonal. In this case the cross-sectional size may be the average cross-sectional extension of the holes.

According to an embodiment, the holes in the mesh are arranged essentially equidistantly in rows. Essentially equidistantly may mean that small variations in the distance between rows may be conceivable. Preferably, the holes in the mesh may be arranged equidistantly in rows. While small deviations may still work, equidistant rows can have the advantage to be easier to manufacture an allow an easier calculation and/or determination of their effects on the conductivity both of signals and of heat on the circuit board. Such an arrangement may be relatively easy to manufacture. Furthermore, equidistant rows may provide a particularly small degradation of signal quality due to the mesh. Preferably, the holes may be arranged according to a regular quadratic grid.

According to an embodiment, the holes are rectangular, preferably quadratic, or circular or hexagonal. These shapes can be advantageous in providing a relatively small degradation of signal quality, when signal lines run across the mesh. A quadratic mesh can be particularly easy to manufacture. While these shapes are preferred, any other shape may in principle be feasible.

According to an embodiment, in the mesh, a fill factor is given by a ratio of holes to conductive material per area being in the range from 30:70 to 80:20, preferably in the range from 40:60 to 60:40, more preferably in the range from 45:55 to 55:45. This ratio can be particularly advantageous in that it can provide a good reduction of induced eddy currents and, at the same time, a low degradation of signal quality.

According to an embodiment, the at least one circuit board further comprises at least one slit cutting at least partially through the conducting layer. The slit or several slits can be an additional measure to reduce the induction of eddy currents. Slits have the advantage that they can be efficient in interrupting eddy currents or increasing the impedance for the eddy currents. On the other hand, a slit may be disadvantageous in that a signal line going across the slit may lead to a significant degradation of signal quality. Hence it may be conceivable to provide one or more slits in between signal lines, such that the signal lines do not cross any slit. At the same time a mesh may be provided in the area of the signal lines. This may be possible, since it has been found that the signal quality is reduced less due to the mesh. Optionally, the at least one slit may be running across the mesh.

According to a further aspect of the invention, a circuit board for an imaging system that is adapted to be placed within the magnetic field created by a magnetic resonance imaging system is provided. The circuit board comprises at least one conducting layer, in particular a copper layer, and a mesh of holes that spreads over at least a partial region of the conducting layer. The circuit board comprises at least one electronic signal line running across the mesh. The circuit board comprises a trace of conducting material running across the mesh and following the course of the at least one signal line, wherein the trace of conducting material is wider than the at least one signal line, such that, at least across the mesh, the at least one signal line is widened by the trace of conducting material. The circuit board may in particular be a circuit board as descried herein. All features and advantages of the imaging system may be adapted to the circuit board and vice versa.

According to a further aspect of the invention, a method for designing a circuit board arrangement comprising at least one circuit board for an imaging system suitable to be placed within a magnetic field produced by a magnetic resonance imaging system is provided. The method comprises the following steps:
(a) setting up different circuit board arrangements, wherein at least some of the different circuit board arrangements comprise different variations of a mesh of holes in at least one conducting layer of the circuit board arrangements, including at least one signal line running across the mesh of holes;
(b) determining a heating of the circuit board due to eddy currents induced by the magnetic field into at least one conducting layer of the at least one circuit board for the different circuit board arrangements when the corresponding circuit board is within the magnetic field of the magnetic resonance imaging system during operation, wherein an impact of the variations of a mesh on the heating are determined;
(c) determining further circuit board properties, including signal integrity and/or wave impedance of signal lines of the circuit board arrangements, wherein an impact of the variations of a mesh on the further circuit board properties are determined;
(d) determining requirements of the further circuit board properties for the imaging system;
(e) based on the determination of heating and the further properties, choosing a circuit board arrangement in order to balance the heating to be as low as possible while the further properties fulfil the requirements of the imaging system.

Optionally, the method may comprise a further step of producing the circuit board that is chosen.

All features and advantages of the imaging system and the circuit board may be adapted to the method and vice versa. The circuit board arrangement may comprise a position an orientation of the circuit board within the imaging system.

For example, the amount of the conducting layer covered by mesh may be different for different circuit board arrangements. For example, one circuit board may have a conducting layer covered completely by a mesh and another circuit board may have a conducting layer having only 50 percent covered by a mesh. For example, the areas of the conducting layer that are covered by mesh may be different for different circuit board arrangements. Hence, the mesh may be provided at different positions for different circuit boards. Some of the circuit board arrangements may comprise further measures to reduce eddy currents, such as slits, a thickness of the conducting layer or and/an orientation of the conducting layer with respect to a magnetic field, the circuit board is planned to be exposed to.

The heating may, for example, be determined by performing a computer simulation of the setup the circuit board is planned to be used in. The simulation may in particular comprise simulating corresponding changing magnetic fields. Additionally, or alternatively, the determining of further circuit board properties may be determined via a computer simulation. The further circuit board properties may be simulated by simulating the electronic circuit of the circuit board. The electronic circuit of the circuit board may be simulated in the context of the imaging system. For example, the electronic circuit of the circuit board may be simulated with respect to a PET system the circuit board is a part of.

Requirements of the signal properties for the imaging system may, for example, be a minimal signal quality and/or integrity. Hence different signal lines may be required to have at least a certain quality for an imaging system to work properly and/or reliably. For example, at least one threshold may be applied that defines the minimum requirements. According to an embodiment, the requirements of the signal properties comprise critical signal lines that require a defined minimum signal integrity. The requirements may for example be determined based on information data that is provided and that lists the requirements.

According to the invention, at least one of the different circuit board arrangements comprises at least one trace of conducting material running across the mesh and following the course of the at least one signal line, wherein the trace of conducting material is wider than the at least one signal line, such that, at least across the mesh, the at least one signal line is widened by the trace of conducting material. Hence the impact of the trace may be determined both with respect to the induced eddy currents and with respect to the signal quality of the corresponding signal line.

The embodiments described herein may be combined with each other unless indicated otherwise. For example,

The accompanying drawings illustrate various exemplary embodiments and methods of various aspects of the invention.
Fig. 1 shows a flow diagram of a method for designing a circuit board arrangement according to an embodiment of the invention;
Fig. 2 shows a medical imaging system according to an embodiment of the invention;
Fig. 3 shows a cross section of a bore of a medical imaging system according to an embodiment of the invention;
Fig. 4 shows a section of a printed circuit board without a mesh;
Fig. 5 shows the same section of the printed circuit board as Fig. 4 but having a mesh of holes according to an embodiment of the invention;
Fig. 6 shows a section of a printed circuit board comprising a slit that is cutting at least partially through the conducting layer
Fig. 7 shows an electronic signal line running across a mesh of a circuit board according to an embodiment of the invention; and
Fig. 8 shows a similar signal line running across a mesh of a circuit board as Fig. 7 but with a trace of conducting material running across the mesh and following the course of the at least one signal line according to an embodiment of the invention.

Similar elements are designated with the same reference signs in the drawings.

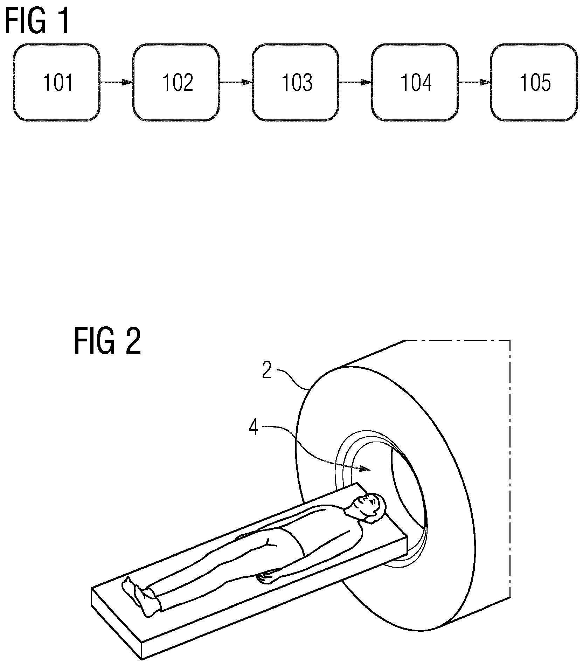

Figure 1 shows a flow diagram of a method for designing a circuit board arrangement according to an embodiment of the invention. The circuit board arrangement comprises at least one circuit board for an imaging system suitable to be placed within a magnetic field produced by a magnetic resonance imaging system. Optionally, the circuit board arrangement may comprise a plurality of circuit boards, e.g. several circuit boards that are part of the electronics of the imaging system. In a first step 101, different circuit board arrangements are set up. At least some of the different circuit board arrangements comprise different variations of a mesh of holes in at least one conducting layer of the corresponding circuit board arrangements. Optionally, one or more signal lines may be running across the mesh of holes. In a further step 102, a heating of the circuit board due to eddy currents induced by the magnetic field into at least one conducting layer of the at least one circuit board is determined for the different circuit board arrangements. Preferably, the impact is simulated when the corresponding circuit board is within the magnetic field of the magnetic resonance imaging system during operation. Therein an impact of the variations of a mesh on the heating are determined. In a further step 103, further circuit board properties are determined. The further circuit board properties include a signal integrity and/or a wave impedance of signal lines of the circuit board arrangements. In particular, an impact of the variations of a mesh on the further circuit board properties are determined. Typically, the mesh may somewhat decrease the signal quality. However, this decrease may be small enough to ignore. On the other hand, in some arrangements the decrease in quality, in particular for very sensible signal lines, may be too great.

Therefore, it is advantageous to determine the actual impact the mesh can have for each circuit board arrangement. In a further step 104 requirements of the further circuit board properties for the imaging system are determined. The requirements of the signal properties may in particular comprise critical signal lines that require a defined minimum signal integrity. In a further step 105, based on the determination of heating and the further circuit board properties, a circuit board arrangement is chosen in order to balance the heating to be as low as possible while the further circuit board properties fulfil the requirements of the imaging system. The chosen circuit board may then be produced.

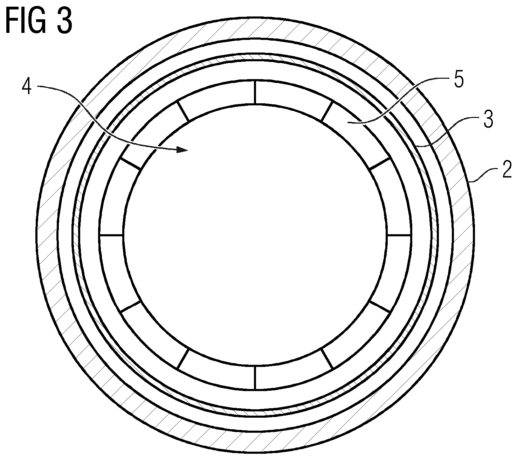

Figure 2 shows a medical imaging system according to an embodiment of the invention. The imaging system comprises a magnetic resonance imaging system with a gradient coil arrangement configured to produce a varying magnetic gradient field during operation. Figure 3 shows a cross section of the bore 2 of the medical imaging system according to an embodiment of the invention. The medical imaging system comprises several printed circuit boards (not shown here) that are arranged in pockets 5 inside the gradient coil 3 of the magnetic resonance imaging system and around an examination area 4. Hence, the printed circuit boards are within the magnetic gradient field of the magnetic resonance imaging system during operation. Each circuit board has at least one conducting layer 6, in particular a copper layer. At least one of the circuit boards comprises a mesh 7 of holes that spreads over at least a partial region of the conducting layer 6. The circuit boards are part of the electronics of a positron emission tomography system that is part of the imaging system.

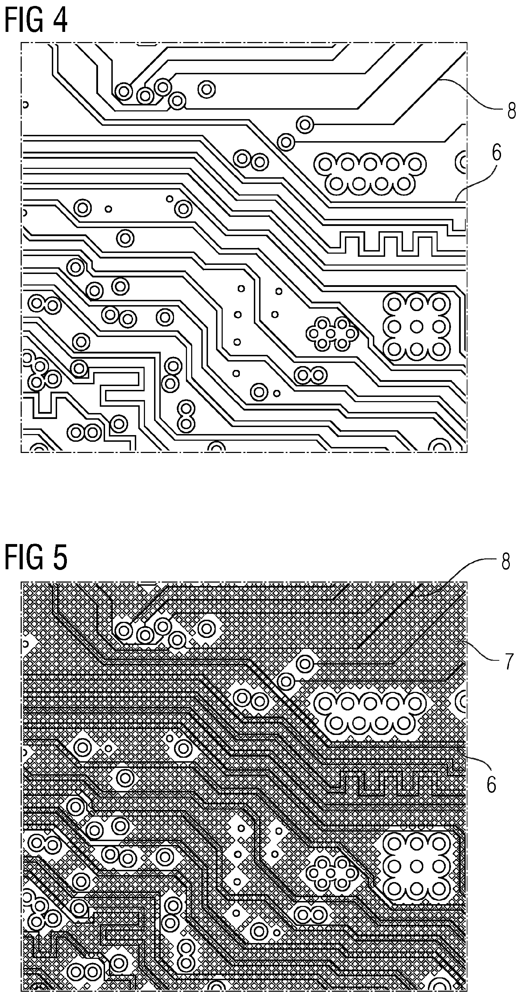

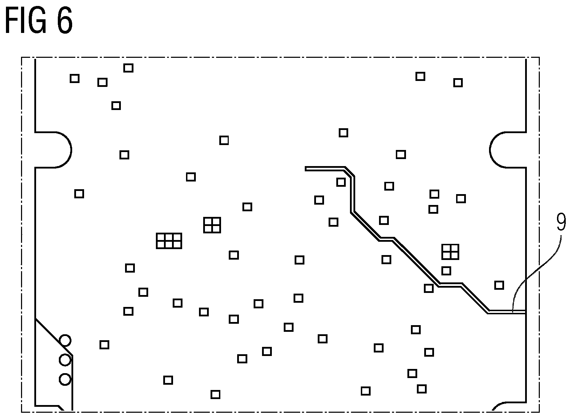

Figure 4 shows a section of a printed circuit board without a mesh 7. Figure 5 shows the same section of the printed circuit board but having a mesh 7 of holes according to an embodiment of the invention. The holes in the mesh are quadratic and arranged equidistantly and in rows. Several electronic signal lines are running across the mesh. The size of the holes, i.e. the side length of the quadrats may preferably be in the range of 100 to 250 micrometres. Figure 6 shows a section of a printed circuit board comprising a slit 9 that is cutting at least partially through the conducting layer. For example, this section or a similar section having a slit 9, may be combined with a section having the mesh 7. Optionally, the slit may also be running across a mesh 7.

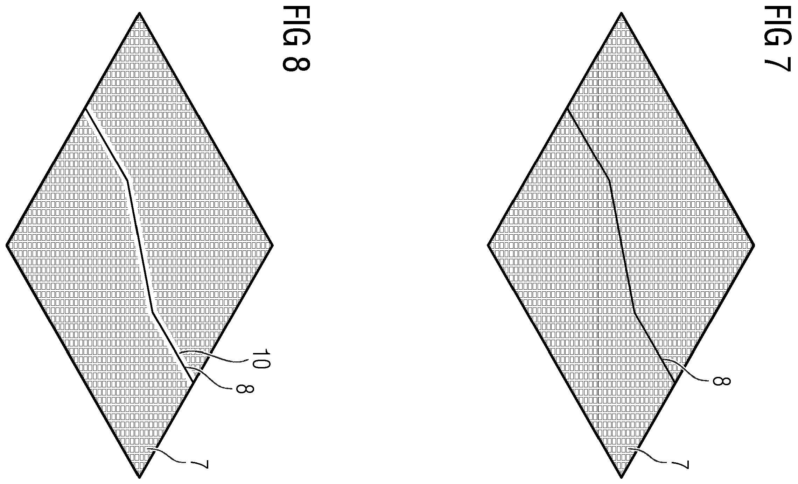

Figure 7 shows an electronic signal line 8 running across a mesh 7 of a circuit board according to an embodiment of the invention. Figure 8 shows a similar signal line 8 running across a mesh 7 of a circuit board according to an embodiment of the invention. However, in this embodiment, the circuit board comprises a trace of conducting material running across the mesh and following the course of the at least one signal line. The trace 10 of conducting material is wider than the signal line 8, such that, across the mesh, the signal line 8 is widened by the trace 10 of conducting material. The signal line may, for example, be a signal line for a clock signal.

## Claims

1. An imaging system, in particular medical imaging system, comprising a magnetic resonance imaging system with a gradient coil (3) arrangement configured to produce a varying magnetic gradient field during operation,
the medical imaging system comprising one or several printed circuit boards arranged such that they are within the magnetic gradient field of the magnetic resonance imaging system during operation, each circuit board having at least one conducting layer (6),
wherein at least one of the circuit boards comprises a mesh (7) of holes that spreads over at least a partial region of the conducting layer (6), wherein the at least one circuit board comprises at least one electronic signal line (8) running across the mesh (7),
**characterized in that**
the at least one circuit board comprises a trace (10) of conducting material running across the mesh (7) and following the course of the at least one signal line (8), wherein the trace (10) of conducting material is wider than the at least one signal line (8), such that, at least across the mesh (7), the at least one signal line (8) is widened by the trace (10) of conducting material.

2. The imaging system according to claim 1,
wherein the imaging system further comprises a positron emission tomography system,
wherein the at least one circuit board is part of the electronics of the positron emission tomography system.

3. The imaging system according to any of the preceding claims,
wherein at least one signal line (8) which is widened by the trace (10) of conducting material is a signal line (8) for a clock signal.

4. The imaging system according to any one of the preceding claims,
wherein the at least one circuit boards comprises a stack of layers.

5. The imaging system according to any one of the preceding claims,
wherein the cross-sectional size of the mesh's (7) holes is 10 to 1000 micrometres, preferably 70 to 500 micrometres, more preferably 100 to 250 micrometres.

6. The imaging system according to any one of the preceding claims,
wherein the holes in the mesh (7) are arranged essentially equidistantly in rows.

7. The imaging system according to any one of the preceding claims,
wherein the holes are, quadratic, circular or hexagonal.

8. The imaging system, according to any one of the preceding claims,
wherein, in the mesh (7), a fill factor is given by a ratio of holes to conductive material per area being in the range from 30:70 to 80:20, preferably in the range from 40:60 to 60:40, more preferably in the range from 45:55 to 55:45.

9. The imaging system according to any one of the preceding claims,
wherein the at least one circuit board further comprises at least one slit (9) cutting at least partially through the conducting layer (6).

10. A circuit board for an imaging system that is adapted to be placed within the magnetic field created by a magnetic resonance imaging system,
wherein the circuit board comprises at least one conducting layer (6) and a mesh (7) of holes that spreads over at least a partial region of the conducting layer (6), wherein the circuit board comprises at least one electronic signal line (8) running across the mesh (7), **characterized in that**
the circuit board comprises a trace (10) of conducting material running across the mesh (7) and following the course of the at least one signal line (8), wherein the trace (10) of conducting material is wider than the at least one signal line (8), such that, at least across the mesh (7), the at least one signal line (8) is widened by the trace (10) of conducting material.

11. A method for designing a circuit board arrangement comprising at least one circuit board for an imaging system suitable to be placed within a magnetic field produced by a magnetic resonance imaging system, the method comprising the following steps:
(a) setting up different circuit board arrangements, wherein at least some of the different circuit board arrangements comprise different variations of a mesh (7) of holes in at least one conducting layer (6) of the circuit board arrangements, including at least one signal line (8) running across the mesh (7) of holes;
(b) determining a heating of the circuit board due to eddy currents induced by the magnetic field into at least one conducting layer (6) of the at least one circuit board for the different circuit board arrangements when the corresponding circuit board is within the magnetic field of the magnetic resonance imaging system during operation, wherein an impact of the variations of a mesh (7) on the heating are determined;
(c) determining further circuit board properties, including signal integrity and/or wave impedance of signal lines (8) of the circuit board arrangements, wherein an impact of the variations of a mesh (7) on the further circuit board properties are determined;
(d) determining requirements of the further circuit board properties for the imaging system;
(e) based on the determination of heating and the further circuit board properties, choosing a circuit board arrangement in order to balance the heating to be as low as possible while the further circuit board properties fulfil the requirements of the imaging system,
**characterized in that**
the at least one of the different circuit board arrangements comprises at least one trace (10) of conducting material running across the mesh (7) and following the course of the at least one signal line (8), wherein the trace (10) of conducting material is wider than the at least one signal line (8), such that, at least across the mesh (7), the at least one signal line (8) is widened by the trace (10) of conducting material.

12. The method according to claim 11,
wherein the requirements of the signal properties comprise critical signal lines (8) that require a defined minimum signal integrity.

## Patentansprüche

1. Abbildungssystem, insbesondere medizinisches Abbildungssystem, umfassend ein Magnetresonanzabbildungssystem mit einer Gradientenspulenanordnung (3), die dazu ausgelegt ist, während des Betriebs ein veränderliches magnetisches Gradientenfeld zu erzeugen,
wobei das medizinische Abbildungssystem eine oder mehrere Leiterplatten umfasst, die so angeordnet sind, dass sie sich während des Betriebs innerhalb des magnetischen Gradientenfelds des Magnetresonanzabbildungssystems befinden, wobei jede Leiterplatte mindestens eine leitende Schicht (6) aufweist,
wobei mindestens eine der Leiterplatten ein Netz (7) aus Löchern umfasst, das sich über mindestens einen Teilbereich der leitenden Schicht (6) erstreckt, wobei die mindestens eine Leiterplatte mindestens eine elektronische Signalleitung (8) umfasst, die über das Netz (7) verläuft,
**dadurch gekennzeichnet, dass**
die mindestens eine Leiterplatte eine Leiterbahn (10) aus leitendem Material umfasst, die über das Netz (7) verläuft und dem Verlauf der mindestens einen Signalleitung (8) folgt, wobei die Leiterbahn (10) aus leitendem Material breiter ist als die mindestens eine Signalleitung (8), so dass zumindest über das Netz (7) hinweg die mindestens eine Signalleitung (8) durch die Leiterbahn (10) aus leitendem Material verbreitert ist.

2. Abbildungssystem nach Anspruch 1,
wobei das Abbildungssystem ferner ein Positronenemissionstomographiesystem umfasst,
wobei die mindestens eine Leiterplatte Teil der Elektronik des Positronenemissionstomographiesystems ist.

3. Abbildungssystem nach einem der vorstehenden Ansprüche, wobei mindestens eine Signalleitung (8), die durch die Leiterbahn (10) aus leitendem Material verbreitert ist, eine Signalleitung (8) für ein Taktsignal ist.

4. Abbildungssystem nach einem der vorstehenden Ansprüche, wobei die mindestens eine Leiterplatte einen Schichtstapel umfasst.

5. Abbildungssystem nach einem der vorstehenden Ansprüche, wobei die Querschnittsgröße der Löcher des Netzes (7) 10 bis 1000 Mikrometer, bevorzugt 70 bis 500 Mikrometer, besonders bevorzugt 100 bis 250 Mikrometer beträgt.

6. Abbildungssystem nach einem der vorstehenden Ansprüche, wobei die Löcher im Netz (7) im Wesentlichen äquidistant in Reihen angeordnet sind.

7. Abbildungssystem nach einem der vorstehenden Ansprüche, wobei die Löcher quadratisch, kreisförmig oder hexagonal sind.

8. Abbildungssystem nach einem der vorstehenden Ansprüche, wobei in dem Netz (7) ein Füllfaktor durch ein Verhältnis von Löchern zu leitendem Material pro Fläche gegeben ist, das im Bereich von 30:70 bis 80:20, bevorzugt im Bereich von 40:60 bis 60:40, besonders bevorzugt im Bereich von 45:55 bis 55:45 liegt.

9. Abbildungssystem nach einem der vorstehenden Ansprüche, wobei die mindestens eine Leiterplatte ferner mindestens einen Schlitz (9) umfasst, der die leitende Schicht (6) mindestens teilweise durchschneidet.

10. Leiterplatte für ein Abbildungssystem, die dazu ausgelegt ist, innerhalb des von einem Magnetresonanzabbildungssystem erzeugten Magnetfelds platziert zu werden,
wobei die Leiterplatte mindestens eine leitende Schicht (6) und ein Netz (7) aus Löchern umfasst, das sich über mindestens einen Teilbereich der leitenden Schicht (6) erstreckt, wobei die Leiterplatte mindestens eine elektronische Signalleitung (8) umfasst, die über das Netz (7) verläuft,
**dadurch gekennzeichnet, dass**
die Leiterplatte eine Leiterbahn (10) aus leitendem Material umfasst, die über das Netz (7) verläuft und dem Verlauf der mindestens einen Signalleitung (8) folgt, wobei die Leiterbahn (10) aus leitendem Material breiter ist als die mindestens eine Signalleitung (8), so dass zumindest über das Netz (7) hinweg die mindestens eine Signalleitung (8) durch die Leiterbahn (10) aus leitendem Material verbreitert ist.

11. Verfahren zum Entwerfen einer Leiterplattenanordnung, die mindestens eine Leiterplatte für ein Abbildungssystem umfasst, die geeignet ist, innerhalb eines von einem Magnetresonanzabbildungssystem erzeugten Magnetfelds platziert zu werden, wobei das Verfahren die folgenden Schritte umfasst:
(a) Einrichten unterschiedlicher Leiterplattenanordnungen, wobei mindestens einige der unterschiedlichen Leiterplattenanordnungen unterschiedliche Variationen eines Netzes (7) aus Löchern in mindestens einer leitenden Schicht (6) der Leiterplattenanordnungen umfassen, einschließlich mindestens einer Signalleitung (8), die über das Netz (7) aus Löchern verläuft;
(b) Bestimmen einer Erwärmung der Leiterplatte aufgrund von Wirbelströmen, die durch das Magnetfeld in mindestens eine leitende Schicht (6) der mindestens einen Leiterplatte induziert werden, für die unterschiedlichen Leiterplattenanordnungen, wenn die entsprechende Leiterplatte während des Betriebs innerhalb des Magnetfelds des Magnetresonanzabbildungssystems ist, wobei eine Auswirkung der Variationen eines Netzes (7) auf die Erwärmung bestimmt wird;
(c) Bestimmen weiterer Leiterplatteneigenschaften, einschließlich Signalintegrität und/oder Wellenimpedanz von Signalleitungen (8) der Leiterplattenanordnungen, wobei eine Auswirkung der Variationen eines Netzes (7) auf die weiteren Leiterplatteneigenschaften bestimmt wird;
(d) Bestimmen von Anforderungen der weiteren Leiterplatteneigenschaften für das Abbildungssystem;
(e) basierend auf der Bestimmung der Erwärmung und der weiteren Leiterplatteneigenschaften, Auswählen einer Leiterplattenanordnung, um die Erwärmung möglichst gering zu halten, während die weiteren Leiterplatteneigenschaften die Anforderungen des Abbildungssystems erfüllen,
**dadurch gekennzeichnet, dass**
die mindestens eine der unterschiedlichen Leiterplattenanordnungen mindestens eine Leiterbahn (10) aus leitendem Material umfasst, die über das Netz (7) verläuft und dem Verlauf der mindestens einen Signalleitung (8) folgt, wobei die Leiterbahn (10) aus leitendem Material breiter ist als die mindestens eine Signalleitung (8), so dass zumindest über das Netz (7) hinweg die mindestens eine Signalleitung (8) durch die Leiterbahn (10) aus leitendem Material verbreitert ist.

12. Verfahren nach Anspruch 11,
wobei die Anforderungen der Signaleigenschaften kritische Signalleitungen (8) umfassen, die eine definierte minimale Signalintegrität erfordern.

## Revendications

1. Un système d'imagerie, en particulier un système d'imagerie médicale, comprenant un système d'imagerie par résonnance magnétique ayant un agencement de bobines (3) à gradient configuré pour produire un champ magnétique à gradient variable pendant le fonctionnement, le système d'imagerie médicale comprenant une ou plusieurs cartes à circuit imprimé agencées de manière à être dans le champ magnétique à gradient du système d'imagerie par résonnance magnétique pendant le fonctionnement, chaque carte à circuit imprimé ayant au moins une couche (6) conductrice,
dans lequel au moins l'une des cartes à circuit imprimé comprend un maillage (7) de trous, qui s'étale sur au moins une région partielle de la couche (6) conductrice, dans lequel la au moins une carte à circuit imprimé comprend au moins une ligne (8) de signal électronique courant dans le maillage (7),
**caractérisé en ce que**
la au moins une carte à circuit imprimé comprend une trace (10) de matériau conducteur courant dans le maillage (7) et suivant le tracé de la au moins une ligne (8) de signal, dans lequel la trace (10) de matériau conducteur est plus large que la au moins une ligne (8) de signal, de manière à ce que, au moins dans le maillage (7), la au moins une ligne (8) de signal soit élargie par la trace (10) de matériau conducteur.

2. Le système d'imagerie suivant la revendication 1,
dans lequel le système d'imagerie comprend en outre un système de tomodensitométrie par émission de positrons, dans lequel la au moins une carte à circuit imprimé fait partie de l'électronique du système de tomodensitométrie par émission de positrons.

3. Le système d'imagerie suivant l'une quelconque des revendications précédentes,
dans lequel au moins une ligne (8) de signal, qui est élargie par la trace (10) de matériau conducteur, est une ligne (8) de signal pour un signal d'horloge.

4. Le système d'imagerie suivant l'une quelconque des revendications précédentes,
dans lequel la au moins une carte à circuit imprimé comprend un empilement de couches.

5. Le système d'imagerie suivant l'une quelconque des revendications précédentes,
dans lequel la dimension en section transversale des trous du maillage (7) va de 10 à 1000 microns, de préférence de 70 à 500 microns, d'une manière encore plus préférée de 100 à 250 microns.

6. Le système d'imagerie suivant l'une quelconque des revendications précédentes,
dans lequel les trous dans le maillage (7) sont disposés sensiblement de manière équidistante en rangées.

7. Le système d'imagerie suivant l'une quelconque des revendications précédentes,
dans lequel les trous sont tétragonaux, circulaires ou hexagonaux.

8. Le système d'imagerie suivant l'une quelconque des revendications précédentes,
dans lequel, dans le maillage (7), un facteur de remplissage est donné par un rapport des trous au matériau conducteur par surface, qui est dans la plage de 30:70 à 80:20, de préférence dans la plage de 40:60 à 60:40, d'une manière plus préférée dans la plage de 45:55 à 55:45.

9. Le système d'imagerie suivant l'une quelconque des revendications précédentes,
dans lequel la au moins une carte à circuit imprimé comprend en outre au moins une fente (9) coupant au moins en partie la couche (6) conductrice.

10. Une carte à circuit imprimé pour un système d'imagerie, qui est propre à être mise dans le champ magnétique créé par un système d'imagerie par résonnance magnétique,
dans laquelle la carte à circuit imprimé comprend au moins une couche (6) conductrice et un maillage (7) de trous, qui s'étale sur au moins une région partielle de la couche (6) conductrice, dans laquelle la carte à circuit imprimé comprend au moins une ligne (8) de signal électronique courant dans le maillage (7),
**caractérisée en ce que**
la carte à circuit imprimé comprend une trace (10) de matériau conducteur courant dans le maillage (7) et suivant le tracé de la au moins une ligne (8) de signal, dans laquelle la trace (10) de matériau conducteur est plus large que la au moins une ligne (8) de signal, de manière à ce que, au moins dans le maillage (7), la au moins une ligne (8) de signal soit élargie par la trace (10) de matériau conducteur.

11. Un procédé de conception d'un agencement de cartes à circuit imprimé comprenant au moins une carte à circuit imprimé pour un système d'imagerie propre à être placé dans un champ magnétique produit par un système d'imagerie par résonnance magnétique, le procédé comprenant les stades suivants :
(a) se procurer différents agencements de cartes à circuit imprimé, dans lequel au moins certains des agencements de cartes à circuit imprimé comprennent des variations différentes de maillage (7) de trous dans au moins une couche (6) conductrice des agencements de cartes à circuit imprimé, comprenant au moins une ligne (8) de signal électronique courant dans le maillage (7) de trous ;
(b) déterminer un chauffage de la carte à circuit imprimé dû à des courants de Foucault induits par le champ magnétique dans au moins une couche (6) conductrice de la au moins une carte à circuit imprimé pour les différents agencements de cartes à circuit imprimé, lorsque la carte à circuit imprimé correspondante est dans le champ magnétique du système d'imagerie par résonnance magnétique pendant le fonctionnement, dans lequel on détermine un impact des variations d'un maillage (7) sur le chauffage ;
(c) déterminer d'autres propriétés de carte à circuit imprimé, incluant l'intégrité du signal et/ou l'impédance d'onde de lignes (8) de signal des agencements de cartes à circuit imprimé, dans lequel on détermine un impact de la variation d'un maillage (7) sur les autres propriétés de carte à circuit imprimé ;
(d) déterminer des exigences des autres propriétés de carte à circuit imprimé pour le système d'imagerie ;
(e) sur la base de la détermination du chauffage et des autres propriétés de carte à circuit imprimé, choisir un agencement de cartes à circuit imprimé afin d'équilibrer le chauffage pour qu'il soit aussi faible que possible, alors que les autres propriétés de carte à circuit imprimé satisfont les exigences du système d'imagerie,
**caractérisé en ce que**
la au moins une carte à circuit imprimé comprend une trace (10) de matériau conducteur courant dans le maillage (7) et suivant le tracé de la au moins une ligne (8) de signal, dans lequel la trace (10) de matériau conducteur est plus large que la au moins une ligne (8) de signal, de manière à ce que, au moins dans le maillage (7), la au moins une ligne (8) de signal soit élargie par la trace (10) de matériau conducteur.

12. Le procédé suivant la revendication 11,
dans lequel les exigences des propriétés du signal comprennent des lignes (8) critiques du signal, qui exigent une intégrité minimum définie du signal.
